# EUROPÄISCHE PATENTSCHRIFT

(11) **EP 1 105 724 B1**
(45) Veröffentlichungstag und Bekanntmachung des Hinweises auf die Patenterteilung: **12.07.2006**
(21) Anmeldenummer: 00936647.7
(22) Anmeldetag: 26.04.2000
(51) Int. Cl.: G01N 33/00

(54) **VORRICHTUNG UND VERFAHREN ZUR DETEKTION VON AMMONIAK**
DEVICE AND METHOD FOR DETECTING AMMONIA
DISPOSITIF ET PROCEDE POUR LA DETECTION DE L'AMMONIAC

(30) Priorität: 29.04.1999 DE 19919472
(43) Veröffentlichungstag der Anmeldung: 13.06.2001
(73) Patentinhaber: ROBERT BOSCH GMBH, 70442 Stuttgart (DE)
(72) Erfinder: STAHL, Roland, D-71691 Freiberg (DE); STROHMAIER, Rainer, D-70563 Stuttgart (DE)
(86) Internationale Anmeldenummer: PCT/DE2000/001297
(87) Internationale Veröffentlichungsnummer: WO 2000/067015

(56) Entgegenhaltungen:
- DE-A- 3 721 572
- US-A- 5 367 875
- US-A- 5 540 047

## Beschreibung

Die Erfindung betrifft eine Vorrichtung und ein Verfahren zur Detektion von Ammoniak, insbesondere eines NH₃-Schlupfes in einer Katalysatoranordnung, nach der Gattung der unabhängigen Ansprüche.

### Stand der Technik

Für die Reduzierung der NOₓ-Emission von mager betriebenen Verbrennungsmotoren, insbesondere von Dieselmotoren, ist es bekannt, zur Abgasnachbehandlung einen SCR-Katalysator ("selective catalytic reduction") einzusetzen. Dazu sei beispielsweise auf D. Schöppe et al., "Ein geregeltes Abgasnachbehandlungssystem zur Erfüllung zukünftiger Emissionsgrenzwerte bei Dieselmotoren", Fortschritt-Berichte, VDI, Reihe 12, Nr. 267, Band 1 (1996), 17. Int. Wiener Motorensymposium, S. 332-353, verwiesen, in dem die Wirkungsweise und der Aufbau eines solchen SCR-Katalysators beschrieben ist.

Zusammenfassend findet in einem SCR-Katalysator eine selektive katalytische Reduktion von NOₓ zu N₂ statt, wobei als Reduktionsmittel NH₃ (Ammoniak) dient, das in bekannter Weise in einem dem SCR-Katalysator vorgeschalteten Hydrolysekatalysator aus einer wäßrigen Harnstofflösung gewonnen werden kann.

Bei Betrieb einer derartigen Anlage zur Abgasnachbehandlung mit einem SCR-Katalysator tritt jedoch das Problem auf, daß zuviel Harnstoff zudosiert wird, so daß nicht umgesetztes Ammoniak über den SCR-Katalysator mit dem Abgas in die Umgebungsluft gelangt. Sofern man diesen NH₃-Schlupf unterdrükken oder überwachen möchte, wird daher ein NH₃-Sensor benötigt.

DE 3721572 beschreibt ein Verfahren zur Steuerung eines Katalysators, der zur Abgasentstickung dient und dem ein Oxidationskatalysator vorgeschaltet ist. Zur optimierten Dosierung von Ammoniak wird am Ausgang des SCR-Katalysators **selektiv** die NO_{X} Konzentration gemessen.

US 5367875 beschreibt eine Anordnung zur Nachbehandlung von Abgasen einer Brennkraftmaschine, bei der einem SCR-Katalysator ein Oxidationskatalysator vorgeschaltet ist. Die Zufuhr von Ammoniak in den Abgastrakt wird ausschließlich über motorintern erfasste Parameter eingestellt.

US 5540047 beschreibt die Verwendung eines SCR-Katalysators zur Abgasnachbehandlung, bei der ein dem Katalysator nachgeordneter Gassensor vorgesehen ist. Dieser Gassensor ist **selektiv** empfindlich für NO_{X} und Ammoniak, und entsprechend der Widerstandskennlinie des Sensors in Abhängigkeit von den Konzentrationen der beiden genannten Gase am Ausgang des Katalysators wird die Menge des dem Katalysator zuzuführenden Harnstoffs eingestellt.

### Vorteile der Erfindung

Die erfindungsgemäße Vorrichtung und das erfindungsgemäße Verfahren mit den kennzeichnenden Merkmalen der unabhängigen Ansprüche hat gegenüber dem Stand der Technik den Vorteil, daß damit die Verwendung aufwendiger, speziell auf NH₃ selektiver Sensoren zum Nachweis von Ammoniak oder eines NH₃₋Schlupfes vermieden wird, die technisch schwierig darzustellen sind. Somit kann vorteilhaft nunmehr auch ein erweitertes Spektrum von Sensorkonzepten oder Sensormaterialien zur Anwendung kommen, d.h. es können auch und insbesondere nicht selektiv auf einzelne oxidierbare Gase oder Gaskomponenten ansprechende, an sich bekannte Gassensoren zum Nachweis für Ammoniak eingesetzt werden, was einen erheblichen Kostenvorteil und eine Vereinfachung hinsichtlich Technologie und Produktion der verwendbaren Gassensoren bedeutet. Insbesondere kann das Spektrum der verwendbaren Gassensoren zum Nachweis von Ammoniak nunmehr sehr vorteilhaft auch auf Gassensoren mit Metallelektroden, speziell auf Pt/Au-Systeme, ausgedehnt werden, die im Stand der Technik bereits als HC-Sensoren verbreitet sind.

Dadurch, daß dem bekannten SCR-Katalysator durch ein vorgeschaltetes erstes Mittel ein bereits zumindest weitgehend, vorzugsweise vollständig, vorgereinigtes oder voroxidiertes Gas oder Abgas zugeführt wird, enthält dieses Gas bereits keine oxidierbaren oder brennbaren Gaskomponenten mehr. Insbesondere sind die Gaskomponenten HC und CO nicht mehr in diesem vorgereinigten Gas enthalten.

Dem derart vorgereinigten Gas wird nun vor der Zufuhr zu dem SCR-Katalysator vorteilhaft über eine zweite Gaszufuhrvorrichtung Ammoniak zugeführt, so daß in dem SCR-Katalysator neben NH₃ kein oxidierbares Gas vorliegt. Falls in dem SCR-Katalysator nun nicht das gesamte zugeführte NH₃ zur Reduktion des vorhandenen NOₓ verbraucht wird, tritt ein NH₃₋Durchbruch oder NH₃-Schlupf in dem SCR-Katalysator auf d.h. das den SCR-Katalysator verlassende Gas enthält Ammoniak. Da jedoch dieses Ammoniak nun die einzige in dem Gas enthaltene oxidierbare Gaskomponente ist, ist es nicht erforderlich, zur Detektion des Ammoniaks ein speziell auf NH₃ selektives Mittel, insbesondere einen speziell auf Ammoniak selektiven Gassensor einzusetzen. Es genügt ein einfacher, allgemein oder breitbandig auf oxidierbare Gaskomponenten ansprechender Sensor, wie er bereits vielfach bekannt ist.

Vorteilhafte Weiterbildungen der Erfindung ergeben sich aus den in den Unteransprüchen genannten Maßnahmen.

So ist es besonders vorteilhaft, wenn das dem SCR-Katalysator zuzuführende Gas vor der Mischung mit Ammoniak in einem an sich bekannten Oxidationskatalysator vorbehandelt wird, der die zumindest weitgehende Oxidation oder Vorreinigung des dem SCR-Katalysator zuzuführenden Gases bewirkt.

Die Zufuhr des Ammoniaks zu dem vorgereinigten oder voroxidierten Gas, das dem SCR-Katalysator zugeführt werden soll und das beispielsweise aus dem Oxidationskatalysator austritt, erfolgt weiter sehr vorteilhaft über eine an sich bekannte Gaszufuhrvorrichtung, die einen Hydrolysekatalysator zur Herstellung von Ammoniak aus einer chemischen Verbindung, insbesondere aus Harnstoff, ein Dosiersystem zur dosierten und kontrollierten Zugabe von Ammoniak zu dem voroxidierten Gas und eine entsprechende Zuleitung umfaßt.

Weiterhin ist es sehr vorteilhaft, wenn das zweite Mittel zum Nachweis der den SCR-Katalysator verlassenden oxidierbaren Gaskomponenten mit einer Verarbeitungseinheit verbunden ist, die über eine Steuereinheit und eine Steuerleitung mit der Gaszufuhrvorrichtung zur Zufuhr von Ammoniak zu dem voroxidierten Gas in Verbindung steht. Damit kann sehr vorteilhaft die Zugabe von Ammoniak zu dem dem SCR-Katalysator zugeführten Gas in Abhängigkeit vom für die Reduktion von NOₓ tatsächlich verbrauchten NH₃ gesteuert bzw. in Form eines geschlossenen Regelkreises derart geregelt werden, daß die Menge des den SCR-Katalysator verlassenden unverbrauchten Ammoniaks minimiert oder auf nahezu Null reduziert wird.

### Zeichnungen

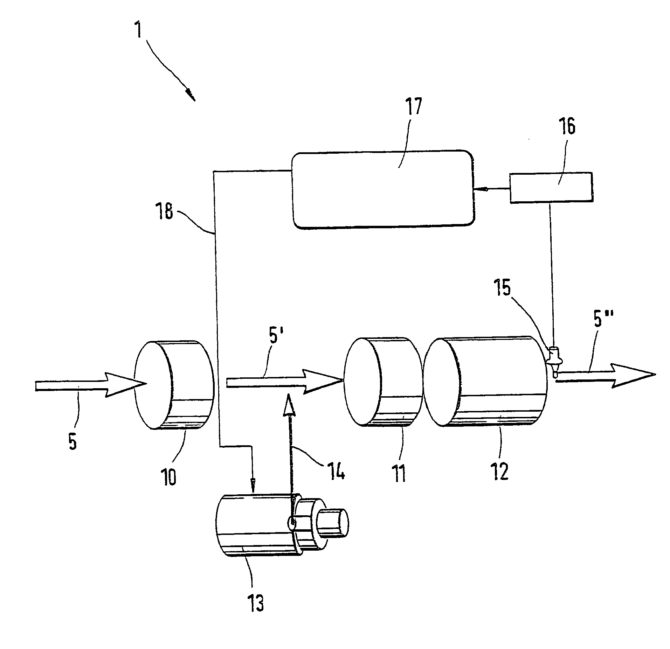

Ausführungsbeispiele der Erfindung werden anhand der Zeichnung und in der nachfolgenden Beschreibung näher erläutert. Die Figur zeigt eine Prinzipskizze einer Katalysatoranordnung zur Detektion eines NH₃-Schlupfes.

### Ausführungsbeispiele

Die Figur zeigt eine Katalysatoranordnung 1, wie sie beispielsweise in der Abgasnachbehandlung von Dieselmotoren oder mager betriebenen Verbrennungsmotoren eingesetzt werden kann. In diese Katalysatoranordnung 1 wird zunächst über entsprechende an sich bekannte Abgasrohre ein zugeführtes Gas 5, insbesondere ein Abgas eines Dieselmotors, eingeleitet, das vielfältige oxidierbare Gaskomponenten, insbesondere beispielsweise HC und CO, sowie außerdem Stickoxide (NOₓ) aufweist. Dieses zugeführte Gas 5 wird zunächst in einen an sich beispielsweise aus der EP 0 800 856 A2 bekannten Oxidationskatalysator 10 eingeleitet, der eine Vorreinigung und Voroxidation des zugeführten Gases 5 vornimmt, so daß die in dem zugefuhrten Gas 5 enthaltenen oxidierbaren Gaskomponenten, insbesondere HC oder CO, weitgehend oder vollständig, beispielsweise zu H₂O oder CO₂, oxidiert oder umgesetzt sind. Aus dem Oxidationskatalysator 10 tritt somit ein zumindest weitgehend oxidiertes Gas 5' aus, das als Schadstoffkomponente lediglich NOₓ enthält.

Dieses zumindest weitgehend oxidierte Gas 5' wird nun im weiteren über eine an sich bekannte erste Gaszufuhrvorrichtung einem SCR-Katalysator 12 zugeführt. Dabei ist jedoch zunächst vorgesehen, dem zumindest weitgehend oxidierten Gas 5' vor dessen Eintritt in den SCR-Katalysator 12 Ammoniak zuzumischen. Dies geschieht mittels einer zweiten Gaszufuhrvorrichtung, die ein Dosiersystem 13, eine Zuleitung 14 und einen Hydrolysekatalysator 11 aufweist. Im einzelnen führt dazu das Dosiersystem 13 über die Zuleitung 14 zunächst dem Hydrolysekatalysator 11 Harnstoff zu, der dort in an sich bekannter Weise zu NH₃ umgesetzt wird. Dieses NH₃ wird dann dem zumindest weitgehend oxidierten Gas 5' zugemischt bevor das so erzeugte Gasgemisch aus NH₃ und dem zumindest weitgehend oxidierten Gas 5' in den SCR-Katalysator 12 gelangt.

Alternativ ist es gemäß der Figur ebenfalls möglich, daß dem zumindest weitgehend oxidierten Gas 5' zunächst über das Dosiersystem 13 und die Zuleitung 14 Harnstoff zudosiert wird, und daß dann diese Mischung dem Hydrolysekatalysator 11 zugeführt wird, der den Harnstoff zu NH₃ umsetzt, so daß das Gasgemisch aus dem zumindest weitgehend oxidiertem Gas 5' und NH₃ entsteht.

In diesem Sinne sind die erste Gaszufuhrvorrichtung und die zweite Gaszufuhrvorrichtung in dem gemäß der Figur erläuterten Ausführungsbeispiel ineinander verwoben oder räumlich ineinander integriert, da die erste Gaszufuhrvorrichtung das erzeugte zumindest weitgehend oxidierte Gas 5' beispielsweise über entsprechende Rohre zunächst der zweiten Gaszufuhrvorrichtung zuführt, die es in dem Hydrolysekatalysator 11 als Teil der zweiten Gaszufuhrvorrichtung durch Zugabe von NH₃ modifiziert und in der somit das zumindest weitgehend oxidierte Gas 5' und das NH₃ vermischt werden. Diese Gasmischung wird dann schließlich dem SCR-Katalysator 12 zugeleitet.

In einer weiteren Variante des erläuterten Ausführungsbeispiels ist es jedoch auch möglich, die erste Gaszufuhrvorrichtung und die zweite Gaszufuhrvorrichtung räumlich voneinander zu trennen, so daß die erste das zumindest weitgehend oxidierte Gas 5' erzeugt und dem SCR-Katalysator direkt zuleitet, und die zweite das Ammoniak erzeugt und ebenfalls direkt dem SCR-Katalysator 12 zuleitet. In dieser Variante wird zweckmäßigerweise zusätzlich das zumindest weitgehend oxidierte Gas 5' und das zugeführte NH₃ vor dem Eintritt in den SCR-Katalysator 12 zuvor über eine geeignete, an sich bekannte Mischvorrichtung vermischt.

In dem SCR-Katalysator 12 werden schließlich mit Hilfe des zugeführten Ammoniaks in an sich bekannter Weise in dem eingeleiteten Gas bzw. Gasgemisch vorhandene Stickoxide (NOₓ) über eine selektive katalytische Reaktion zu N₂ reduziert. Im Anschluß an den SCR-Katalysator 12 wird das dort austretende Gas 5'' dann weitergeleitet und abgeführt.

Das austretende Gas 5'' weist nunmehr als Schadstoffkomponenten lediglich NOₓ auf, falls die Zugabe von Ammoniak über die zweite Gaszufuhrvorrichtung nicht ausreichend war (unzureichende Schadstoffreduktion), oder NH₃ auf, falls die Zugabe von Ammoniak zu groß war (NH₃-Schlupf oder NH₃₋Durchbruch). Weitere oxidierbare Gaskomponenten, wie CO und HC wurden bereits mittels des Oxidationskatalysators 10 aus dem zugeführten Gas 5 entfernt.

Um daher quantitativ zu überprüfen, ob die NH₃-Zufuhr angemessen, zu groß oder zu gering war, ist nach dem SCR-Katalysator 12 ein Gassensor 15 vorgesehen, der nichtselektiv bzw. breitbandig auf oxidierbare Gaskomponenten empfindlich ist und der mit dem austretenden Gas 5'' in Kontakt ist. Der Gassensor 15 ist beispielsweise ein an sich bekannter Gassensor mit Metallelektroden auf Pt/Au-Basis, die als HC-Sensoren weit verbreitet sind. Derartige Sensoren weisen eine logarithmische Kennlinie als Funktion der Gaskonzentration auf d.h. sie besitzen eine sehr hohe Empfindlichkeit speziell im Bereich von Konzentrationen unter 100 ppm, wie sie bei einem NH₃-Durchbruch typischerweise auftreten, und erlauben daher sehr gut und einfach eine quantitative Bestimmung der oxidierbaren Gaskomponenten in dem austretenden Gas 5''.

Der Gassensor 15 steht weiterhin mit einer an sich bekannten Verarbeitungseinheit 16 zur Aufnahme, Auswertung und Aufbereitung der Signale des Gassensors 15 in Verbindung. Die Verarbeitungseinheit 16 ist daneben mit einer Steuereinheit 17 verbunden, die über eine Steuerleitung 18 mit dem Dosiersystem 13 in Verbindung ist. Dabei kann die Verarbeitungseinheit 16, die Steuereinheit 17, die Steuerleitung 18 und das Dosiersystem 13 oder die zweite Gaszufuhrvorrichtung auch in einem Bauteil integriert sein.

Über die Steuereinheit 17 ist somit in Abhängigkeit vom Meßsignal des Gassensors 15 eine Steuerung der Ammoniakzufuhr zu dem weitgehend oxidierten Gas 5' vor dessen Eintritt in den SCR-Katalysator 12 möglich, wobei in an sich bekannter Weise in Form eines geschlossenen Regelkreises die Ammoniakzufuhr stets derart geregelt wird, daß einerseits eine möglichst vollständige Reduktion und damit Entfernung von Stickoxiden aus dem austretenden Gas 5'' erzielt wird, daß andererseits aber gleichzeitig möglichst kein Ammoniak in dem austretenden Gas 5'' enthalten ist.

### Bezugszeichenliste

- 1: Katalysatoranordnung

- 5: zugeführtes Gas
- 5': weitgehend oxidiertes Gas
- 5'': austretendes Gas

- 10: Oxidationskatalysator
- 11: Hydrolysekatalysator
- 12: SCR-Katalysator
- 13: Dosiersystem
- 14: Zuleitung
- 15: Gassensor
- 16: Verarbeitungseinheit
- 17: Steuereinheit
- 18: Steuerleitung

## Patentansprüche

1. Vorrichtung zur Detektion von Ammoniak, insbesondere eines NH₃-Schlupfes in einer Katalysatoranordnung (1), mit einem SCR-Katalysator (12), dem über eine erste Zufuhrvorrichtung ein Gas, insbesondere ein Abgas eines mager betriebenen Verbrennungsmotors, und über eine zweite Zufuhrvorrichtung Ammoniak zuführbar ist, **dadurch gekennzeichnet, dass** ein erstes Mittel vorgesehen ist, das vor der Zufuhr des Gases zu dem SCR-Katalysator (12) in dem Gas enthaltene oxidierbare Gaskomponenten zumindest weitgehend oxidiert, und dass ein zweites Mittel vorgesehen ist, das ein aus dem SCR-Katalysator (12) austretendes Gas (5") hinsichtlich enthaltener oxidierbarer Komponenten analysiert, wobei mit dem zweiten Mittel nichtselektiv oxidierbare Gase oder Gaskomponenten nachweisbar sind,

2. Vorrichtung nach Anspruch 1, **dadurch gekennzeichnet, dass** das erste Mittel ein Oxidationskatalysator (10) ist.

3. Vorrichtung nach Anspruch 1, **dadurch gekennzeichnet, dass** das zweite Mittel ein Gassensor (15) ist.

4. Vorrichtung nach Anspruch 1, **dadurch gekennzeichnet, dass** die zweite Zufuhrvorrichtung einen Hydrolysekatalysator (11), ein Dosiersystem (13) und eine Zuleitung (14) aufweist.

5. Vorrichtung nach Anspruch 4, **dadurch gekennzeichnet, dass** mit dem Dosiersystem (13) und der Zuleitung (14) dem Hydrolysekatalysator (11) eine chemische Verbindung, insbesondere Harnstoff, zuführbar ist.

6. Vorrichtung nach Anspruch 5, **dadurch gekennzeichnet, dass** aus der chemischen Verbindung in dem Hydrolysekatalysator (11) Ammoniak freisetzbar ist.

7. Vorrichtung nach Anspruch 1, **dadurch gekennzeichnet, dass** die erste Zufuhrvorrichtung das dem SCR-Katalysator (12) zuzuführende, bereits zumindest weitgehend oxidierte Gas (5') zunächst der zweiten Zufuhrvorrichtung zuführt.

8. Vorrichtung nach Anspruch 7, **dadurch gekennzeichnet, dass** in der zweiten Zufuhreinrichtung das dem SCR-Katalysator (12) zuzuführende Ammoniak mit dem über die erste Zufuhreinrichtung dem SCR-Katalysator (12) zuzuführenden oxidierten Gas (5') mischbar ist.

9. Vorrichtung nach Anspruch 1, **dadurch gekennzeichnet, dass** das zweite Mittel, insbesondere der Gassensor (15), mit einer Verarbeitungseinheit (16) verbunden ist, die über eine Steuereinheit (17) und eine Steuerleitung (18) mit der zweiten Zufuhrvorrichtung in Verbindung steht.

10. Vorrichtung nach Anspruch 9, **dadurch gekennzeichnet, dass** die Verarbeitungseinheit (16), die Steuereinheit (17) und die zweite Zufuhrvorrichtung in einem Bauteil integriert sind.

11. Vorrichtung nach Anspruch 9, **dadurch gekennzeichnet, dass** über ein Mess-Signal des zweiten Mittels die zugeführte Menge an Ammoniak durch die zweite Zufuhrvorrichtung regelbar ist.

12. Verfahren zur Detektion von Ammoniak, insbesondere eines NH₃-Schlupfes in einer Katalysatoranordnung (1), mit einem SCR-Katalysator (12), dem über eine erste Zufuhrvorrichtung ein Gas, insbesondere ein Abgas eines mager betriebenen Verbrennungsmotors, und über eine zweite Zufuhrvorrichtung Ammoniak zugeführt werden, **dadurch gekennzeichnet, dass** vor der Zufuhr des Gases zu dem SCR-Katalysator (12) in dem Gas enthaltene oxidierbare Gaskomponenten zunächst zumindest weitgehend oxidiert werden, und dass danach das aus dem SCR-Katalysator (12) austretende Gas (5") hinsichtlich enthaltener oxidierbarer Komponenten analysiert wird, wobei nichtselektiv die Konzentration der oxidierbaren Komponenten des austretenden Gases (5") bestimmt wird.

13. Verfahren nach Anspruch 12, **dadurch gekennzeichnet, dass** die Oxidation des Gases vor der Zufuhr zu dem SCR-Katalysator (12) über einen Oxidationskatalysator (10) erfolgt.

14. Verfahren nach Anspruch 12, **dadurch gekennzeichnet, dass** die Analyse des austretenden Gases (5") mit Hilfe eines Gassensors (15) vorgenommen wird.

15. Verfahren nach Anspruch 14, **dadurch gekennzeichnet, dass** der Gassensor (15) mit der zweiten Zufuhrvorrichtung in Verbindung steht und diese hinsichtlich der dem SCR-Katalysator (12) zugeführten Menge an Ammoniak derart regelt, dass die Konzentration der oxidierbaren Komponenten des aus dem SCR-Katalysator (12) austretenden Gases (5") minimiert wird.

16. Verwendung der Vorrichtung oder des Verfahrens nach mindestens einem der vorangehenden Ansprüche zum Nachweis und/oder zur Minimierung eines NH₃₋Schlupfes in einer Katalysatoranordnung zur Reduktion von NOₓ-Emission von Verbrennungsmaschinen.

## Claims

1. Device for detecting ammonia, in particular an NH₃ slippage in a catalytic converter arrangement (1), having an SCR catalytic converter (12), to which a gas, in particular an exhaust gas from a lean-burn internal combustion engine, can be fed via a first feed device, and to which ammonia can be fed via a second feed device, **characterized in that** a first means is provided, which at least substantially oxidizes oxidizable gas components contained in the gas before the gas is fed to the SCR catalytic converter (12), and **in that** a second means is provided, which analyses a gas (5'') emerging from the SCR catalytic converter (12) for its content of oxidizable components, it being possible to use the second means to detect oxidizable gases or gas components non-selectively.

2. Device according to Claim 1, **characterized in that** the first means is an oxidation catalytic converter (10).

3. Device according to Claim 1, **characterized in that** the second means is a gas sensor (15).

4. Device according to Claim 1, **characterized in that** the second feed device has a hydrolysis catalytic converter (11), a metering system (13) and a feed line (14).

5. Device according to Claim 4, **characterized in that** a chemical compound, in particular urea, can be fed to the hydrolysis catalytic converter (11) using the metering system (13) and the feed line (14).

6. Device according to Claim 5, **characterized in that** ammonia can be released from the chemical compound in the hydrolysis catalytic converter (11).

7. Device according to Claim 1, **characterized in that** the first feed device first of all feeds the gas (5') which is to be fed to the SCR catalytic converter (12) and has already been at least substantially oxidized to the second feed device.

8. Device according to Claim 7, **characterized in that** the ammonia that is to be fed to the SCR catalytic converter (12) can be mixed in the second feed device with the oxidized gas (5') that is to be fed to the SCR catalytic converter (12) via the first feed device.

9. Device according to Claim 1, **characterized in that** the second means, in particular the gas sensor (15), is connected to a processing unit (16), which via a control unit (17) and a control line (18) is connected to the second feed device.

10. Device according to Claim 9, **characterized in that** the processing unit (16), the control unit (17) and the second feed device are integrated in one component.

11. Device according to Claim 9, **characterized in that** the quantity of ammonia supplied by the second feed device can be controlled using a measurement signal from the second means.

12. Method for detecting ammonia, in particular an NH₃ slippage in a catalytic converter arrangement (1), having an SCR catalytic converter (12), to which a gas, in particular an exhaust gas from a lean-burn internal combustion engine, is fed via a first feed device, and to which ammonia is fed via a second feed device, **characterized in that** before the gas is fed to the SCR catalytic converter (12), oxidizable gas components contained in the gas are first of all at least substantially oxidized, and **in that** thereafter the gas (5'') which emerges from the SCR catalytic converter (12) is analysed for its content of oxidizable components, the concentration of the oxidizable components in the emerging gas (5'') being determined non-selectively.

13. Method according to Claim 12, **characterized in that** the oxidation of the gas is effected by means of an oxidation catalytic converter (10) before the gas is fed to the SCR catalytic converter (12).

14. Method according to Claim 12, **characterized in that** the analysis of the emerging gas (5'') is carried out with the aid of a gas sensor (15).

15. Method according to Claim 14, **characterized in that** the gas sensor (15) is connected to the second feed device and controls the latter with regard to the quantity of ammonia fed to the SCR catalytic converter (12) in such a manner that the concentration of the oxidizable components in the gas (5'') emerging from the SCR catalytic converter (12) is minimized.

16. Use of the device or method according to at least one of the preceding claims for detecting and/or minimizing an NH₃ slippage in a catalytic converter arrangement for reducing NOₓ emissions from internal combustion engines.

## Revendications

1. Dispositif de détection d'ammoniac, en particulier d'une fuite de NH₃ dans un système de catalyseurs (1), avec un catalyseur à réduction catalytique sélective (12) recevant un gaz, en particulier un gaz d'échappement d'un moteur à combustion fonctionnant par mélange pauvre, peut être amené par un premier dispositif d'amenée et de l'ammoniac par un deuxième dispositif d'amenée,
**caractérisé par**
un premier moyen qui, avant l'amenée du gaz au catalyseur à réduction catalytique sélective (12), oxyde au moins largement les composants gazeux oxydables contenus dans le gaz, et un deuxième moyen qui analyse les composants oxydables contenus, du gaz (5") sortant du catalyseur à réduction catalytique sélective (12), le deuxième moyen permettant de détecter de manière non sélective des gaz ou des composants gazeux oxydables.

2. Dispositif selon la revendication 1,
**caractérisé en ce que**
le premier moyen est un catalyseur d'oxydation (10).

3. Dispositif selon la revendication 1,
**caractérisé en ce que**
le deuxième moyen est un capteur de gaz (15).

4. Dispositif selon la revendication 1,
**caractérisé en ce que**
le deuxième dispositif d'amenée présente un catalyseur d'hydrolyse (11), un système de dosage (13) et une conduite d'amenée (14).

5. Dispositif selon la revendication 4,
**caractérisé en ce qu'**
un composé chimique, en particulier de l'urée, peut être amené par le système de dosage (13) et la conduite d'amenée (14) au catalyseur d'hydrolyse (11).

6. Dispositif selon la revendication 5,
**caractérisé en ce que**
de l'ammoniac peut être dégagé à partir du composé chimique dans le catalyseur d'hydrolyse (11).

7. Dispositif selon la revendication 1,
**caractérisé en ce que**
le premier dispositif d'amenée amène tout d'abord au deuxième dispositif d'amenée le gaz (5') à amener au catalyseur à réduction catalytique sélective (12), déjà au moins largement oxydé.

8. Dispositif selon la revendication 7,
**caractérisé en ce que**
dans le deuxième dispositif d'amenée, l'ammoniac à amener au catalyseur à réduction catalytique sélective (12) est miscible au gaz (5') oxydé à amener au catalyseur à réduction catalytique sélective (12) par le premier dispositif d'amenée.

9. Dispositif selon la revendication 1,
**caractérisé en ce que**
le deuxième moyen, en particulier le capteur de gaz (15), est relié à une unité de traitement (16) qui est en liaison avec le deuxième dispositif d'amenée par l'intermédiaire d'une unité de commande (17) et d'une conduite de commande (18).

10. Dispositif selon la revendication 9,
**caractérisé en ce que**
l'unité de traitement (16), l'unité de commande et le deuxième dispositif d'amenée sont intégrés en un composant.

11. Dispositif selon la revendication 9,
**caractérisé en ce que**
la quantité d'ammoniac amenée à travers le deuxième dispositif d'amenée est réglable au moyen d'un signal de mesure du deuxième moyen.

12. Procédé pour la détection de l'ammoniac, en particulier d'une fuite de NH₃ dans un système de catalyseurs (1), avec un catalyseur à réduction catalytique sélective (12) auquel un gaz, en particulier un gaz d'échappement d'un moteur à combustion fonctionnant par mélange pauvre, peut être amené par un premier dispositif d'amenée et de l'ammoniac par un deuxième dispositif d'amenée,
**caractérisé en ce qu'**
avant l'amenée du gaz au catalyseur à réduction catalytique sélective (12), des composants gazeux oxydables contenus dans le gaz sont tout d'abord au moins largement oxydés et ensuite le gaz (5") sortant du catalyseur à réduction catalytique sélective (12) est analysé en ce qui concerne les composants oxydables contenus, la concentration des composants oxydables du gaz sortant (5") étant déterminée de manière non sélective.

13. Procédé selon la revendication 12,
**caractérisé en ce que**
l'oxydation du gaz a lieu avant l'amenée au catalyseur à réduction catalytique sélective (12) par un catalyseur d'oxydation (10).

14. Procédé selon la revendication 12,
**caractérisé en ce que**
l'analyse du gaz sortant (5") est effectuée à l'aide d'un capteur de gaz (15).

15. Procédé selon la revendication 14,
**caractérisé en ce que**
le capteur de gaz (15) est en liaison avec le deuxième dispositif d'amenée et régule celle-ci en ce qui concerne la quantité d'ammoniac amenée au catalyseur à réduction catalytique sélective (12), de sorte que la concentration des composants oxydables du gaz (5") sortant du catalyseur à réduction catalytique sélective (12) est réduite au minimum.

16. Utilisation du dispositif ou du procédé selon au moins l'une quelconque des revendications précédentes, pour détecter et/ou réduire au minimum une fuite de NH₃ dans un système de catalyseurs destiné à réduire l'émission de NOₓ de moteurs à combustion.
